(19) Europäisches Patentamt
European Patent Office
Office européen des brevets

(11) Publication number: 0 474 874 B1

(12) # EUROPEAN PATENT SPECIFICATION

(45) Date of publication of patent specification: **18.10.95**

(51) Int. Cl.[6]: **A61K 31/195**

(21) Application number: **91903819.0**

(22) Date of filing: **08.02.91**

(86) International application number:
**PCT/JP91/00200**

(87) International publication number:
**WO 91/11997 (22.08.91 91/19)**

(54) **Maillard Reaction inhibitor compositions.**

(30) Priority: **19.02.90 JP 39241/90**

(43) Date of publication of application:
**18.03.92 Bulletin 92/12**

(45) Publication of the grant of the patent:
**18.10.95 Bulletin 95/42**

(84) Designated Contracting States:
**AT BE CH DE DK ES FR GB GR IT LI LU NL SE**

(56) References cited:
**JP-A- 0 567 747**

(73) Proprietor: **Senju Pharmaceutical Co., Ltd.**
**5-8, Hiranomachi 2-chome,**
**Chuo-Ku**
**Osaka-shi,**
**Osaka 541 (JP)**

(72) Inventor: **INOUE, Jun**
**13-57, Shojaku 4-chome,**
**Settsu-shi**
**Osaka 566 (JP)**

(74) Representative: **Kinzebach, Werner, Dr. et al**
**Patentanwälte**
**Reitstötter, Kinzebach und Partner**
**Postfach 86 06 49**
**D-81633 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 474 874 B1

## Description

### Technical Field

This invention relates to pharmaceutical compositions comprising compounds capable of inhibiting the denaturation reaction of proteins by reductive sugars such as glucose, which is known by the name of Maillard's reaction. More specifically this invention relates to pharmaceutical compositions comprising compounds capable of inhibiting the formation of Amadori rearrangement products which originate from non-enzymatic bond formation between glucose and proteins. Furthermore, this invention relates to the use of such compounds for preparing a pharmaceutical composition for the treatment of disorders developing via Maillard's reaction.

### Background Art

The reaction in which proteins turn brown by reacting non-enzymatically with reductive sugars such as glucose (hereinafter referred to as "the glycosylation") was first reported by Maillard in 1912 [Maillard, L.C., Compt. Rend. Soc. Biol., 72:599 (1912).] Since then, the reaction has been widely recognized by the name of Maillard's reaction in the field of food chemistry. For example, it has been noted that proteins react with glucose in stored or heated food, generate a brown color and finally are denaturated by formation of cross-linkings among molecules.

Later, attention was directed to reactions of glucose with proteins which may occur in living bodies when Rahbar reported that the level of $Hb_{A1c}$, a minor component of hemoglobin, was found elevated in red blood cells of diabetic patients [Rahbar, S., Clin. Chim. Acta, 22:296 (1968).] And, through structural analysis of $Hb_{A1c}$, it has been confirmed that Maillard's reaction occurs in living bodies.

The mechanism of Maillard's reaction in living bodies has been presented by Brownlee et al. [Brownlee, M. et al., Science, 232:1629 (1986).] The reaction proceeds as follows.

At first, the aldehyde group of the open-ring structure of glucose reacts with an amino group in protein molecule to form a Schiff's base. The resulting Schiff's base is unstable and is rapidly converted non-enzymatically into Amadori rearrangement product via intra-molecular rearrangement reaction. If this protein is maintained for a long period of time within the body, the rearranged product undergoes a gradual dehydration reaction to form a new glucose derivative. This derivative then irreversively forms cross-linkings with a variety of molecules including proteins to form bridges among molecules, thus yielding aggregation products of, chiefly, proteins.

This type of product resulting from advanced reactions of glycosylated proteins is usually abbreviated to AGE (Advanced Glycosylation End product.)

In parallel to the formation of AGE, biological adaptability of the protein is lowered, and the protein becomes less soluble and more resistant to proteases and, in many cases, turns yellow-brown and becomes fluorescent.

Though also observed in healthy human, Maillard's reaction is markedly noted in those with diabetes mellitus, which is characterized by the elevation of blood glucose. Maillard's reaction is especially notable in proteins with a slower rate of metabolic turnover, for example crystallins, which are the structural proteins in the lens, and collagens. While a variety of disorders, for example neuropathy, cataract, nephropathy, retinopathy, arthrosclerosis and atherosclerosis, are noted as complications of diabetes mellitus, these disorders bear a very close resemblance with disorders noted quite frequently in the aged human.

It, therefore, is regarded that AGE is also formed gradually from proteins with a slower turnover rate by glycosylation with glucose even at a nomal level of blood sugar.

With this background, efforts have been made to find compounds which may inhibit Maillard's reaction within living bodies. An example of such efforts has been shown by Brownlee as cited who reported that aminoguanidine inhibits Maillard's reaction in vitro and suppresses AGE formation in arterial walls of diabetic rats in vivo. In Japanese Patent Publication Kokai No. 142114/87, it has been suggested that aminoguanidine, $\alpha$-hydrazinohistidine and lysine may block the active carbonyl group of Amadori rearrangement products to inhibit AGE formation. It has also been disclosed that different compounds may suppress Maillard's reaction. Such compounds include thiosemicarbazides, 1,3-diaminoguanidine and benzoylhydrazine (Japanese Patent Publication Kokai No. 56614/89), and various derivatives of guanidine (Japanese Patent Publication Kokai No. 83059/89.)

In the patent publications cited above, researches for inhibitors of Maillard's reaction were made using the amount of AGE, the end product of Maillard's reaction, as an index. The present inventor, instead, took the inhibition of formation of Amadori rearrangement product as an index in the investigation. This was

based on an estimation that a markedly effective inhibition of Maillard's reaction may be expected by inhibiting the very formation of Amadori rearrangement product, which is the immediate causing factor in protein aggregation process in Maillard's reaction.

Bruggemann et al. [J. Bruggemann et al., Lebensm. Unters. Forsch., 137:137-143 (1968)] and Finot et al. [P.A. Finot et al., Experientia, 24:1097-1099 (1968)] have reported that the amount of ε-N-(furoyl-methyl)-L-lysine (hereinafter referred to as "furosine"), which is an Amadori rearrangement product resulted from non-enzymatic glycosylation of ε-amino residue of lysine in proteins, may be taken as an index of the non-enzymatic glycosylation of protein molecules. The present inventor made an intensive research for the optimal experimental condition for formation of furosine from protein dissolved in water containing glucose, and, according to the condition thus established, evaluated various compounds for the presence and strength of inhibitory effect on furosine formation.

Previously, the utilization of the aminobenzoic acid derivative para-aminosalicylic acid (PAS) in the treatment of a patient suffering from diabetes mellitus was reported by Dandona et al., Postgraduate Medical Journal 56 (1980), pp. 135-136. This report shows that the patient developed hypoglycaemic coma upon treatment with PAS. This effect is discussed as probably being exerted through a mechanism similar to that of aspirin in pancreatectomized animals, viz. induction of a fall in blood glucose concentration through an increase in the peripheral uptake of glucose by tissues with a concomitant suppression of fatty acid release.

Now, the present inventor discovered that some derivatives of aminobenzoic acids have a potent inhibitory effect on furosine formation.

Disclosure of Invention

Thus the present invention comprises a pharmaceutical composition for inhibition of Maillard's reaction characterized in that it contains a compound selected from

5-hydroxyanthranilic acid,
3-hydroxyanthranilic acid,
4-nitroanthranilic acid,
3-aminosalicylic acid or
3-amino-4-hydroxybenzoic acid.

The present invention also comprises the use of a compound of formula (I)

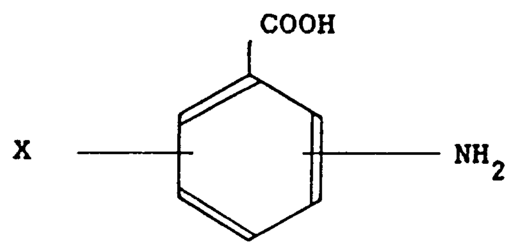

(I)

wherein X denotes a hydroxyl group or a nitro group, a pharmaceutically acceptable ester thereof or a pharmaceutically acceptable salt of the said compound or the said ester for the preparation of a pharmaceutical composition for the treatment or prophylaxis of disorders developing via Maillard's reaction, where the disorders are selected from the diabetic complications coronary heart disease, peripheral circulation disorders, cerebrovascular disorders, neuropathy, nephropathy, arterio-sclerosis, arthrosclerosis, cataract and retinopathy or the age-associated disorders atherosclerosis, coronary heart disease, cerebrovascular disorders and senile cataract.

Examples of the pharmaceutically acceptable esters of the Maillard's reaction inhibitor compound (I) include lower alkyl esters of the carboxyl group of the compound such as methyl ester, ethyl ester, n-propyl ester and isopropyl ester, and esters of the phenolic hydroxyl group of the compound such as esters with lower carboxylic acids including acetic acid ester, oxalic acid ester, malonic acid ester, maleic acid ester and succinic acid ester, and esters with inorganic acids including phosphoric acid ester.

Examples of suitable salts of the compound (I) or pharmaceutically acceptable esters thereof include, in particular, alkali metal salts thereof such as sodium salt and potassium salt, alkaline earth metal salts thereof such as calcium salt and magnesium salt, and salts thereof with inorganic acids such as hydrochloric acid, sulfuric acid and phosphoric acid, or with organic acids such as acetic acid and maleic

acid.

Salts which are usually accepted as pharmaceuticals are included in the scope of the present invention.

The inhibitors of Maillard's reaction of the present invention may be administered orally or non-orally. For non-oral administration, the inhibitors may be administered parenterally for systemic purpose or topically, for example, in the form of eye drops.

The Maillard's reaction inhibitor of the present invention may be administered orally at a dose - as the compound (I) - of, generally, 1 to 1,000 mg/day, more preferably 5 to 200 mg/day. For injection, the dose may be generally 0.1 to 100 mg/day, more preferably 1 to 50 mg/day.

For eye drops, it may be applied in the form of liquid at a concentration of, generally, 0.05 to 5.0 w/v %, more preferably 0.1 to 2.0 w/v %.

However, the examples above are not intended to limit the dose range. A suitable dose may be set according to the type and severity of disorders and schedules of treatment in each case.

The Maillard's reaction inhibitor of the present invention may be formed into, for example, tablets, pilles, powder, granules or capsules for oral administration, aqueous or non-aqueous solution, suspension or emulsion for injection, or eye drops or eye ointment for ophthalmic topical use.

For preparing pharmaceutical composition of the present invention into the form of tablets for oral administration, ingredients usually incorporated in tablet preparation may suitably be utilized.

Such ingredients include, for example, diluent bases such as hydroxypropylcellulose, crystalline cellulose, corn starch, polyvinylpyrrolidone and magnesium metasilicate aluminate, lubricants such as magnesium stearate, disintegrators such as fibrinous calcium gluconate, and solubilizers such as glutamic acid and aspartic acid.

For preparing a pharmaceutical composition of the present invention in the form of aqueous injection, ingredients usually incorporated in injectable preparations may suitably be utilized. Such ingredients include, for example, buffering agents such as phosphates, preservatives such as chlorobutanol, stabilizers such as sodium sulfite, and isotonizers such as sodium chloride.

For preparing a pharmaceutical composition of the present invention into the form of eye drops, ingredients usually incorporated in the formation of eye drops may suitably utilized. Such ingredients include, for example, buffering agents such as phosphates, borates, acetates and citrates, preservatives such as chlorobutanol, methylparaben, propylparaben, benzalkonium chloride and chlorhexidine digluconate, stabilizers such as sodium sulfite, sodium bisulfite and sodium edetate, isotonizers such as sodium chloride, potassium chloride, mannitol, sorbitol and glycerol, and solubilizers such as polysorbate 80 and cyclodextrins.

(Pharmacological test)

The effect of the Maillard's reaction inhibitors of the present invention was determined as follows using the test compounds listed below.

They are known compounds and were purchased from the market.

AB-1: 5-hydroxyanthranilic acid
AB-2: 3-hydroxyanthranilic acid
AB-3: 4-nitroanthranilic acid
AB-4: 5-aminosalicylic acid
AB-5: 4-aminosalicylic acid
AB-6: 3-aminosalicylic acid
AB-7: 3-amino-4-hydroxybenzoic acid

(Test methods)

Sample solutions as shown below were aseptically prepared from bovine serum albumine (No. A-8022, Sigma)(hereinafter referred to as BSA), 50 mM phosphate buffer solution (pH 7.3) and the test compounds listed in Table 1 and aminoguanidine.

The sample solutions were kept for 4 weeks at 37 °C, and the amount of furosine which was formed by non-enzymatic glycosylation was determined by HPLC according to the method of Schleicher et al. [J. Clin. Biochem., 19:81-87 (1981).] Thus, the sample solutions after reaction were dialyzed, and aliquots of 1 ml were lyophylized and then hydrolyzed by the addition of 1 ml of 6 N hydrochloric acid followed by heating at 100 °C for 20 hours. After removal of hydrochloric acid by evaporation, 1 ml of water was added to each sample, and the samples were subjected to filtration using a filter with the pore size of 0.45 µm. The filtrate was used as the sample for HPLC. ODS-120T (Tosoh Corporation) was used for the column and 7 mM

phosphoric acid solution was used as the eluant. The absorbance peak whose ratio of peak area at 280 mm/254 mm was 3.9/1 was regarded as the peak corresponding to furosine.

[Constituents in the phosphate buffer solution]

| | |
|---|---|
| Normal sample; | 20 mg/ml BSA |
| Control sample; | 20 mg/ml BSA and 50 mM glucose |
| Test sample; | 20 mg/ml BSA, 50 mM glucose and 5 mM test compound |

Upon the area of the peak of furosine of each sample, the inhibition rate of furosine formation by the test compound was calculated as follows.

$$\text{Inhibition rate (\%)} = (c\text{-}d) \div (c\text{-}n) \times 100$$

c; peak area of furosine of the control sample
d; peak area of furosine of the test sample
n; peak area of furosine of the normal sample

(Results)

As shown in Table 1, each of the test compounds, AB-1 to AB-7, exhibited a remarkably potent inhibitory effect in comparison with aminoguanidine, a known inhibitor of Maillard's reaction.

Table 1

| Test compound | Inhibition rate (%) |
|---|---|
| AB-1 | 94.1 |
| AB-2 | 69.4 |
| AB-3 | 47.6 |
| AB-4 | 50.7 |
| AB-5 | 70.0 |
| AB-6 | 53.4 |
| AB-7 | 60.4 |
| aminoguanidine | 8.0 |

## Best Mode for Carrying out the Invention

Examples:

The following are examples of pharmaceutical compositions of Maillard's reaction inhibitors of the present invention. Each code in the formulae represents each of the compounds described in the section of Pharmacological test.

(Example 1) Oral tablets

According to the formula below, the ingredients are formed into a tablet by a conventional method. Sugar coating may optionally be made.

| | |
|---|---|
| AB-1 | 100 mg |
| lactose | 80 mg |
| corn starch | 17 mg |
| magnesium stearate | 3 mg |

(Example 2) Oral tablets

According to the formula below, the ingredients are formed into a tablet by a conventional method. Sugar coating may optionally be made.

| | |
|---|---|
| AB-2 | 50 mg |
| corn starch | 90 mg |
| lactose | 30 mg |
| hydroxypropylcellulose | 25 mg |
| magnesium stearate | 5 mg |

(Example 3) Capsules

According to the formula below, the ingredients are admixed and granulated by a conventional method and filled in capsules in an amount of 100 mg/capsule.

| | |
|---|---|
| AB-3 | 10 mg |
| corn starch | 45 mg |
| lactose | 20 mg |
| crystalline cellulose | 24 mg |
| talc | 0.5 mg |
| magnesium stearate | 0.5 mg |

(Example 4) Injection

According to the formula below, the ingredients are admixed by a conventional method to dissolve. The solution is filtered, filled into vials and autoclaved to sterilize.

| | |
|---|---|
| AB-4 | 20 mg |
| chlorobutanol | 5 mg |
| water for injection | 1 ml |

(Example 5) Eye drops

According to the formula below, the ingredients are admixed by a conventional method to dissolve, and the solution is sterilized by filtration.

| | |
|---|---|
| AB-5 | 0.5 g |
| boric acid | 1.0 g |
| borax | q.s.(pH 7.0) |
| sodium chloride | 0.25 g |
| disodium edetate | 0.02 g |
| chlorobutanol | 0.2 g |
| polysorbate 80 | 0.2 g |
| sodium sulfite | 0.2 g |
| sterile purified water | to 100 ml |

(Example 6) Eye ointment

According to the formula below, the ingredients are admixed by a conventional method to form an eye ointment.

| | |
|---|---|
| AB-7<br>white vaseline | 0.5 g<br>100 g |

Industrial Applicability

The inhibitors of Maillard's reaction represented by the formula (I) and pharmaceutically acceptable salts thereof, inhibit the very formation of Amadori rearrangement product, the immediate causing factor of cross linkings among protein molecules.

The pharmaceutical compositions of the present invention, accordingly, may be useful for treatment and prophylaxis of diabetic complications, for example coronary heart disease, peripheral circulation disorders, cerebrovascular disorders, neuropathy, nephropathy, arteriosclerosis, arthrosclerosis, cataract and retinopathy, and age-associated disorders such as atherosclerosis, coronary heart disease, cerebrovascular disorders and senile cataract.

**Claims**

**Claims for the following Contracting States : AT, BE, CH, DE, DK, FR, GB, IT, LI, LU, NL, SE**

**1.** Pharmaceutical composition comprising a compound selected from
5-hydroxyanthranilic acid,
3-hydroxyanthranilic acid,
4-nitroanthranilic acid,
3-aminosalicylic acid or
3-amino-4-hydroxybenzoic acid.

**2.** Use of a compound of formula (I),

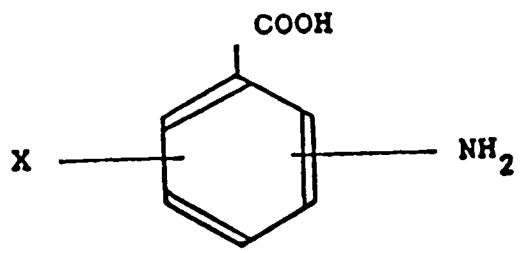

(I)

wherein X denotes a hydroxyl group or a nitro group, a pharmaceutically acceptable ester thereof or a pharmaceutically acceptable salt of the said compound or the said ester, for the preparation of a pharmaceutical composition for the treatment or prophylaxis of disorders developing via Maillard's reaction, where the disorders are selected from the diabetic complications coronary heart disease, peripheral circulation disorders, cerebrovascular disorders, neuropathy, nephropathy, arteriosclerosis, arthrosclerosis, cataract and retinopathy or the age-associated disorders atherosclerosis, coronary heart disease, cerebrovascular disorders and senile cataract.

**3.** Use according to claim 2, wherein said compound is selected from:
5-hydroxyanthranilic acid,
3-hydroxyanthranilic acid,
4-nitroanthranilic acid,
5-aminosalicylic acid,
4-aminosalicylic acid,
3-aminosalicylic acid or

3-amino-4-hydroxybenzoic acid.

**Claims for the following Contracting States : ES, GR**

1. A process for preparing a pharmaceutical composition which comprises providing a compound selected from
5-hydroxyanthranilic acid,
3-hydroxyanthranilic acid,
4-nitroanthranilic acid,
3-aminosalicylic acid or
3-amino-4-hydroxybenzoic acid,
in a form suitable for administration.

2. Use of a compound of formula (I),

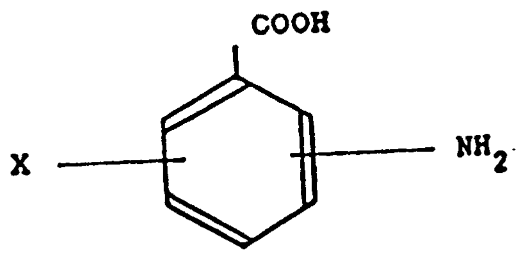

(I)

wherein X denotes a hydroxyl group or a nitro group, a pharmaceutically acceptable ester thereof or a pharmaceutically acceptable salt of the said compound or the said ester, for the preparation of a pharmaceutical composition for the treatment or prophylaxis of disorders developing via Maillard's reaction, where the disorders are selected from the diabetic complications coronary heart disease, peripheral circulation disorders, cerebrovascular disorders, neuropathy, nephropathy, arteriosclerosis, arthrosclerosis, cataract and retinopathy or the age-associated disorders atherosclerosis, coronary heart disease, cerebrovascular disorders and senile cataract.

3. Use according to claim 2, wherein said compound is selected from:
5-hydroxyanthranilic acid,
3-hydroxyanthranilic acid,
4-nitroanthranilic acid,
5-aminosalicylic acid,
4-aminosalicylic acid,
3-aminosalicylic acid or
3-amino-4-hydroxybenzoic acid.

**Patentansprüche**
**Patentansprüche für folgende Vertragsstaaten : AT, BE, CH, DE, DK, FR, GB, IT, LI, LU, NL, SE**

1. Pharmazeutisches Mittel, welches einen Stoff umfaßt, der ausgewählt ist unter
5-Hydroxyanthranilsäure,
3-Hydroxyanthranilsäure,
4-Nitroanthranilsäure,
3-Aminosalicylsäure oder
3-Amino-4-hydroxybenzoesäure.

2. Verwendung einer Verbindung der Formel (I)

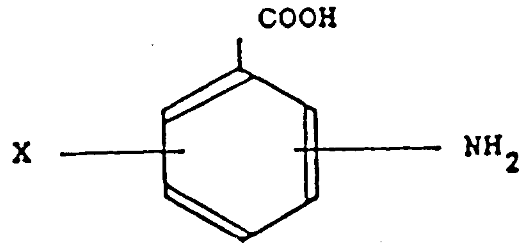

(I)

worin X eine Hydroxylgruppe oder Nitrogruppe bedeutet, eines pharmazeutisch verträglichen Esters davon oder eines pharmazeutisch verträglichen Salzes dieser Verbindung oder dieses Esters, zur Herstellung eines pharmazeutischen Mittels zur Behandlung oder Prophylaxe von Erkrankungen, die via Maillard Reaktion entstehen, wobei die Erkrankungen unter den diabetischen Komplikationen koronare Herzerkrankung, periphere Zirkulationserkrankungen, cerebrovaskuläre Erkrankungen, Neuropathie, Nephropathie, Arteriosklerose, Arthosklerose, Katarakt und Retinopathie oder den altersbedingten Erkrankungen Atherosklerose, koronare Herzerkrankung, cerebrovaskuläre Erkrankungen und Alterskatarakt ausgewählt sind.

3. Verwendung nach Anspruch 2, wobei dieser Stoff ausgewählt ist unter
5-Hydroxyanthranilsäure,
3-Hydroxyanthranilsäure,
4-Nitroanthranilsäure,
5-Aminosalicylsäure,
4-Aminosalicylsäure,
3-Aminosalicylsäure oder
3-Amino-4-hydroxybenzoesäure.

**Patentansprüche für folgende Vertragsstaaten : ES, GR**

1. Verfahren zur Herstellung eines pharmazeutischen Mittels, welches die Bereitstellung eines Stoffes, der ausgewählt ist unter
5-Hydroxyanthranilsäure,
3-Hydroxyanthranilsäure,
4-Nitroanthranilsäure,
3-Aminosalicylsäure oder
3-Amino-4-hydroxybenzoesäure,
in einer zur Verabreichung geeigneten Form umfaßt.

2. Verwendung einer Verbindung der Formel (I)

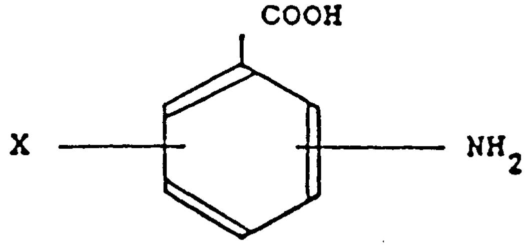

(I)

worin X eine Hydroxylgruppe oder Nitrogruppe bedeutet, eines pharmazeutisch verträglichen Esters davon oder eines pharmazeutisch verträglichen Salzes dieser Verbindung oder dieses Esters, zur Herstellung eines pharmazeutischen Mittels zur Behandlung oder Prophylaxe von Erkrankungen, die via Maillard's Reaktion entstehen, wobei die Erkrankungen unter den diabetischen Komplikationen koronare Herzerkrankung, periphere Zirkulationserkrankungen, cerebrovaskuläre Erkrankungen, Neuropathie,

Nephropathie, Arteriosklerose, Arthosklerose, Katarakt und Retinopathie oder den altersbedingten Erkrankungen Atherosklerose, koronare Herzerkrankung, cerebrovaskuläre Erkrankungen und Alterskatarakt ausgewählt sind.

**3.** Verwendung nach Anspruch 2, wobei dieser Stoff ausgewählt ist unter
5-Hydroxyanthranilsäure,
3-Hydroxyanthranilsäure,
4-Nitroanthranilsäure,
5-Aminosalicylsäure,
4-Aminosalicylsäure,
3-Aminosalicylsäure oder
3-Amino-4-hydroxybenzoesäure.

**Revendications**

**Revendications pour les Etats contractants suivants : AT, BE, CH, DE, DK, FR, GB, IT, LI, LU, NL, SE**

**1.** Composition pharmaceutique comprenant un composé choisi parmi :
l'acide 5-hydroxyanthranilique,
l'acide 3-hydroxyanthranilique,
l'acide 4-nitroanthranilique,
l'acide 3-aminosalicylique ou
l'acide 3-amino-4-hydroxybenzoïque,
sous une forme convenant pour l'administration.

**2.** Utilisation d'un composé répondant à la formule (I) :

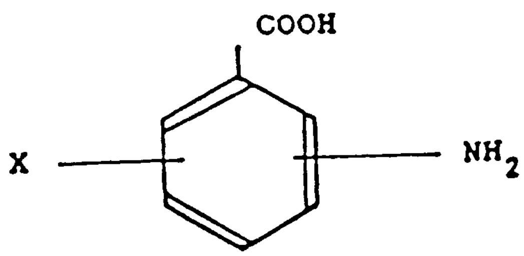

(I)

dans laquelle X désigne un groupe hydroxyle ou un groupe nitro, un de ses esters pharmaceutiquement acceptables ou un sel pharmaceutiquement acceptable de ce composé ou de cet ester, pour la préparation d'une composition pharmaceutique pour le traitement ou la prophylaxie de troubles provenant de la réaction de Maillard, dans laquelle les troubles sont choisis parmi des complications diabétiques telles que l'insuffisance coronarienne, les troubles de la circulation périphérique, les troubles cérébrovasculaires, la neuropathie, la néphropathie, l'artériosclérose, l'arthrosclérose, la cataracte et la rétinopathie ou parmi des troubles associés à l'âge tels que l'athérosclérose, l'insuffisance coronarienne, les troubles cérébrovasculaires et la cataracte sénile.

**3.** Utilisation selon la revendication 2, dans laquelle ce composé est choisi parmi :
l'acide 5-hydroxyanthranilique,
l'acide 3-hydroxyanthranilique,
l'acide 4-nitroanthranilique,
l'acide 5-aminosalicylique,
l'acide 4-aminosalicylique,
l'acide 3-aminosalicylique ou
l'acide 3-amino-4-hydroxybenzoïque.

**Revendications pour les Etats contractants suivants : ES, GR**

**1.** Procédé de préparation d'une composition pharmaceutique qui comprend le fait d'amener un composé choisi parmi :

l'acide 5-hydroxyanthranilique,
l'acide 3-hydroxyanthranilique,
l'acide 4-nitroanthranilique,
l'acide 3-aminosalicylique ou
l'acide 3-amino-4-hydroxybenzoïque,
sous une forme convenant pour l'administration.

2. Utilisation d'un composé répondant à la formule (I) :

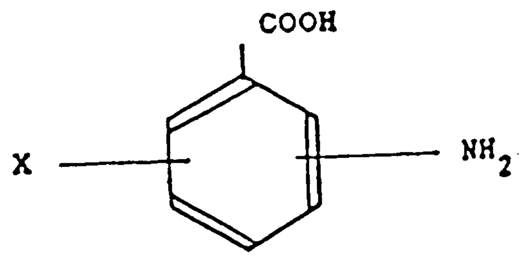

(I)

dans laquelle X désigne un groupe hydroxyle ou un groupe nitro, un de ses esters pharmaceutiquement acceptables ou un sel pharmaceutiquement acceptable de ce composé ou de cet ester, pour la préparation d'une composition pharmaceutique pour le traitement ou la prophylaxie de troubles provenant de la réaction de Maillard, dans laquelle les troubles sont choisis parmi des complications diabétiques telles que l'insuffisance coronarienne, les troubles de la circulation périphérique, les troubles cérébrovasculaires, la neuropathie, la néphropathie, l'artériosclérose, l'arthrosclérose, la cataracte et la rétinopathie ou parmi des troubles associés à l'âge tels que l'athérosclérose, l'insuffisance coronarienne, les troubles cérébrovasculaires et la cataracte sénile.

3. Utilisation selon la revendication 2, dans laquelle ce composé est choisi parmi :
l'acide 5-hydroxyanthranilique,
l'acide 3-hydroxyanthranilique,
l'acide 4-nitroanthranilique,
l'acide 5-aminosalicylique,
l'acide 4-aminosalicylique,
l'acide 3-aminosalicylique ou
l'acide 3-amino-4-hydroxybenzoïque.